Europäisches Patentamt

(19) European Patent Office

Office européen des brevets

(11) EP 0 786 470 A1

# (12) EUROPEAN PATENT APPLICATION
published in accordance with Art. 158(3) EPC

(43) Date of publication:
30.07.1997 Bulletin 1997/31

(21) Application number: 94924995.7

(22) Date of filing: 24.08.1994

(51) Int. Cl.$^6$: **C07K 5/027**, A61K 38/05

(86) International application number:
PCT/JP94/01394

(87) International publication number:
WO 95/06060 (02.03.1995 Gazette 1995/10)

(84) Designated Contracting States:
DE FR GB

(30) Priority: 24.08.1993 JP 209766/93

(71) Applicant: SEIKAGAKU KOGYO KABUSHIKI KAISHA
Tokyo 103 (JP)

(72) Inventors:
• WATANABE, Yasuo
Nerima-ku, Tokyo 176 (JP)
• SAKURAI, Katsukiyo
Higashiyamato-shi, Tokyo 207 (JP)
• HORI, Yusuke
Tokyo 167 (JP)

(74) Representative: Hansen, Bernd, Dr.Dipl.-Chem. et al
Hoffmann, Eitle & Partner
Patentanwälte
Postfach 81 04 20
81904 München (DE)

## (54) PEPTIDE COMPOUND

(57) The invention aims at providing a novel peptide compound which can readily pass the hemoencephalic barrier and has a high hypoexcitability effect as compared with γ-aminobutyric acid and a high regioselectivity as compared with the convention γ-aminobutyric acid derivatives. The invention relates to a peptide compound represented by the general formula R-CO-(L or D)-Ser-X-GABA (wherein R-CO represents alkylcarbonyl, alkoxycarbonyl or an amino-protecting group in peptide chemistry; (L or D)-Ser represents a (L or D)-serine residue; GABA represents a γ-aminobutyric acid residue; and X represents a glycine or L-leucine residue), a salt thereof, and a central nervous system depressant containing the same as the active ingredient.

EP 0 786 470 A1

## Description

TECHNICAL FIELD

This invention relates to a novel peptide compound, more particularly to a peptide compound which has psychotropic actions, especially antispasmodic action.

BACKGROUND ART

It is known that $\gamma$-aminobutyric acid is an amino acid which exists in the central nervous system as an inhibitory neurotransmitter and inhibits nervous excitation at various sites in the nervous system. It has been indicated by recent studies that necrosis of nerve cells is induced as a result of the release of excess excitatory amino acids caused by transient cerebral ischemia, but progress of the necrosis is inhibited by the application of $\gamma$-aminobutyric acid. Therefore, $\gamma$-aminobutyric acid is greatly expected as a cerebral function- and central nerve-related drug.

On the other hand, Piv-Leu-GABA which shows antispasmodic action (*J. Neurochem.*, vol.42, No.6, 1984, pp.1762 - 1766) and (Gly- or Leu- or Ser-)-GABA that shows central nervous action (JP-A-Sho.62-270552) are known as $\gamma$-aminobutyric acid derivatives (Piv represents pivaloyl group, Gly represents glycine residue, Leu represents leucine residue, Ser represents serine residue and GABA represents $\gamma$-aminobutyric acid residue).

However, $\gamma$-aminobutyric acid has disadvantages in that it does not pass through the blood-brain barrier and its activity is reduced due to its extremely short half-life.

The present invention aims at providing a novel peptide compound which can easily pass through the blood-brain barrier, has higher excitation inhibitory effect in comparison with $\gamma$-aminobutyric acid and also has higher site selectivity in comparison with the prior $\gamma$-aminobutyric acid derivatives.

DISCLOSURE OF THE INVENTION

On the assumption that particularly L-Ser and D-Ser will enhance the action of $\gamma$-aminobutyric acid, because the former is one of the central nervous inhibiting amino acids and the latter has more strong central nervous system depressing action, and that, since Ser-Gly is a terminal structure of proteoglycan, a glycosaminoglycan chain will be synthesized and added to the compound administered into the brain to show such functions as to inhibit $\gamma$-aminobutyric acid decomposition and nervous elongation, the inventors of the present invention have conducted intensive studies and accomplished the present invention by finding a peptide compound which can easily pass through the blood-brain barrier, has higher excitation inhibiting effect in comparison with $\gamma$-aminobutyric acid and also has higher site selectivity in comparison with the above-described $\gamma$-aminobutyric acid derivatives.

The present invention relates to a peptide compound represented by the formula (1) and salts thereof.

R-CO-(L or D)-Ser-X-GABA        (1)

wherein R-CO represents an alkylcarbonyl group, an alkoxycarbonyl group or an amino group-protecting group used in peptide chemistry; (L or D)-Ser represents an (L or D)-serine residue; GABA represents a $\gamma$-aminobutyric acid residue; and X represents a glycine residue or an L-leucine residue.

The present invention also relates to a central nerve depressant which comprises a peptide compound represented by the above formula (1) or a pharmaceutically acceptable salt thereof as an active ingredient.

The structure of formula (1) is a structure of tripeptide derivative in which the amino group of Ser is linked to R-CO by acid amide bond, the carboxyl group of Ser is linked to the amino group of X by acid amide bond and the carboxyl group of X is linked to the amino group of GABA by acid amide bond. In this specification, amino acids or derivatives thereof which have D- or L-form are all in the L-form unless whether they are in the D- or L-form is not particularly indicated. The group represented by R-CO is a group which binds to the amino group of L- or D-serine, and its examples include those which are usually used as protecting groups of the amino group. The alkyl group or alkoxy group represented by R is not particularly limited, but preferably a group which renders passage of the compound of the formula (1) through the blood-brain barrier easier. Examples of such a group include branched or straight chain alkyl or alkoxy groups having about 1 to about 20 carbon atoms. Illustrative examples of R-CO include pivaloyl, isobutyryl, isovaleryl, tert-amyloxycarbonyl, t-butoxycarbonyl, benzyloxycarbonyl, ethoxycarbonyl and the like groups. Particularly preferred are branched alkylcarbonyl or alkoxycarbonyl groups which include pivaloyl, isobutyryl, isovaleryl, tert-amyloxycarbonyl and t-butoxycarbonyl groups. Introduction of the group represented by R-CO can be effected by known techniques. For example, it may be effected by allowing an acid halide represented by R-CO-Hal (Hal means chlorine or the like halogen atom) or an acid anhydride represented by R-CO-O-CO-R to react with the amino group of L- or D-serine.

According to the present invention, certain amino acids, peptides, protecting groups, solvents and the like are shown by abbreviations in accordance with the rules of International Union of Pure and Applied Chemistry (IUPAC) or International Union of Biochemistry (IUB) or by symbols commonly used in this field, such as the following examples.

Boc     t-butoxycarbonyl
Z       benzyloxycarbonyl
HONSu   N-hydroxysuccinimide
TFA     trifluoroacetic acid
THF     tetrahydrofuran
NMM     N-methylmorpholine
DCC     dicyclohexylcarbodiimide

The term site selectivity as used herein means selective action on caudate nucleus putamen where the presence of γ-aminobutyric acid nervous system has been revealed.

The peptide compound represented by the above formula (1) includes its free form and optional salt form, and particularly preferred examples of the salt include pharmaceutically acceptable salts. Illustrative examples of such salts include salts with alkali metals such as lithium, sodium, potassium and the like, alkaline earth metals such as calcium, magnesium and the like and other metals such as aluminum and the like.

Production method of the compound of the present invention is not particularly limited, and it can be produced in accordance with known techniques.

For example, it can be synthesized by a solid phase or liquid phase method in accordance with the method described in "The Peptides" (vol.1 (1965), E. Schröder and K. Lübke, Academic Press, New York, U.S.A.) or "Fundamentals and Experiments of Peptide Synthesis" (Izumiya et al., Maruzen, 1985). Examples of the condensation method for the formation of peptide bond include an azide method, an acid chloride method, an acid anhydride method, a mixed acid anhydride method, a DCC method, active ester methods (a p-nitrophenyl ester method, an N-hydroxysuccinimide ester method, a cyanomethyl ester method and the like), a method in which Woodward reagent is used, a carbonyldiimidazole method, an oxidation reduction method, DCC-additive (HONB (N-hydroxy-5-norbornene-2,3-dicarboxylic acid imide), HOBt (1-hydroxybenztriazole), HONSu) methods and the like, and these methods may be used alone or as an optional combination thereof as occasion demands.

In the reaction step for the synthesis of the peptide of the above formula (1), a functional group which should not take part in the reaction is protected by a usual protecting group, and the protecting group is eliminated after completion of the reaction.

Examples of the deprotection method include a catalytic reduction method, a liquid hydrogen fluoride (hydrofluoric acid) method and an acid treatment method using sulfonic acids, 2~4 M hydrochloric acid/dioxane, TFA, hydrogen bromide/acetic acid or formic acid and the like.

In addition, a functional group which takes part in the reaction, such as carboxyl group, is generally activated. Each of these reaction methods is well known, and reagents and the like to be used therein can also selected optionally from known substances.

Illustrative examples of the amino group-protecting group to be used in the synthetic reaction step include Z, Boc, tert-amyloxycarbonyl, isobornyloxycarbonyl, p-methoxybenzyloxycarbonyl, 2-chloro-benzyloxycarbonyl, adamantyloxycarbonyl, trifluoroacetyl, phthalyl, formyl, o-nitrophenylsulfenyl, 3-nitro-2-pyridinesulfenyl, 9-fluorenylmethyloxycarbonyl, diphenylphosphinothioyl and the like.

Illustrative examples of the carboxyl group-protecting group to be used in the synthetic reaction step include methyl ester, ethyl ester, propyl ester, butyl ester, tert-butyl ester, benzyl ester, p-nitrobenzyl ester, p-chlorobenzyl ester, benzhydryl ester, phenacyl ester, carbobenzoxy hydrazide, tert-butyloxycarbonyl hydrazide, trityl hydrazide and the like.

Examples of the activated carboxyl group include corresponding acid chloride, acid anhydride or mixed acid anhydride, azide, active ester (ester with pentachlorophenol, p-nitrophenol, N-hydroxysuccinimide, N-hydroxybenztriazole or N-hydroxy-5-norbornene-2,3-dicarboxyimide for example) and the like.

In some cases, the peptide bond forming reaction can be carried out in the presence of a condensing agent which includes for example a carbodiimide reagent of a water soluble carbodiimide such as DCC, 1-ethyl-3-(3-dimethylaminopropyl)carbodiimide hydrochloride (EDC) or the like, carbonyldiimidazole, benztriazole-N-hydroxytrisdimethylaminophosphoniumhexafluorophosphide, diphenylphosphorylazide, tetraethyl pyrophosphate or the like.

In general, the peptide compound of the formula (1) is produced in accordance with the above-described polypeptide synthesis method, for example by a so-called stepwise method in which amino acids are sequentially condensed one by one to the carboxyl terminal amino acid.

The condensation reaction can be carried out generally at a temperature of from -10°C to 60°C within a period of from 1 to 24 hours.

The following describes an example of the synthesis of the compound of the formula (1) by a liquid phase stepwise method. The carboxyl group of an α-N-protected amino acid represented by $R^1$-X, wherein $R^1$ represents a protecting group of the amino group and X has the same meaning as in the above formula (1), is activated using for example HONSu and DCC, the resulting active ester is allowed to react with γ-aminobutyric acid to form acid amide bond (-CONH-) and then the thus synthesized $R^1$-X-GABA is converted into X-GABA by eliminating the amino group-protecting group. After protecting the amino group of L- or D-serine, the carboxyl group is activated to form an active ester. The active ester is allowed to react with X-GABA to produce $R^1$-(L or D)-Ser-X-GABA through the formation of acid amide bond. Next, the amino group-protecting group of this compound is eliminated, and the thus deprotected compound is allowed to react with a carboxylic acid represented by RCOOH (pivalic acid for example) or a reactive derivative thereof (an acid halide for example), thereby introducing an acyl group and synthesizing R-CO-(L or D)-Ser-X-GABA represented by the formula (1). In this connection, each of the synthesized intermediates obtained in the above-described synthesis steps are purified by extraction with an appropriate solvent such as ethyl acetate or the like to be subjected to the next synthetic reaction step.

More particularly, the peptide compound of the present invention represented by Piv-Ser-Leu-GABA can be obtained through condensation of amino acids by an active ester method, for example in the following manner.

After synthesis of an active ester of Boc-Leu using HONSu and DCC, the carboxyl group of Leu and the amino group of γ-aminobutyric acid are allowed to condense, and the product is purified by extraction with ethyl acetate and deprotected by eliminating Boc with TFA, thereby obtaining an intermediate. An active ester of Boc-Ser is added to the amino terminal amino group of the intermediate to effect its condensation with the amino group. After carrying out purification and deprotection in the same manner, Ser-Leu-GABA is obtained and then pivaloyl chloride is linked to the amino group of Ser to obtain the desired compound. Also, Piv-Ser-Gly-GABA can be synthesized in the same manner.

The present invention also provide a central nervous system depressant which comprises at least one of the peptide compound represented by the formula (1) and pharmaceutically acceptable salts thereof as an active ingredient.

Physiological activities of the peptide compound of the present invention can be determined by measuring spontaneous behavior numbers, brain wave, pressurized weight in accordance with modified Randall-Selitt method and the like of rats. Physiological saline containing 13% ethanol may be used as a control, and γ-aminobutyric acid and Piv-Leu-GABA (referred to as PLG hereinafter) may be used as comparative drugs.

The peptide compound of the present invention can be formulated into an oral or parenteral preparation together with carriers, fillers and other additives to serve as a central nervous system depressant.

Examples of the oral preparation include solid preparations such as powders, granules, capsules, tablets and the like and liquid preparations such as syrups, elixirs, emulsions and the like.

Examples of the parenteral preparation include injections, rectal administration preparations, pessaries, external preparations for skin use, inhalations, aerosols, ophthalmic solutions and the like.

Its dose can be optionally decided depending on the age, health condition, body weight and the like of each patient, and is within the range of generally from 5 ng to 200 mg/kg, preferably from 10 ng to 100 mg/kg once or several divided doses per day.

## BRIEF DESCRIPTION OF THE DRAWINGS

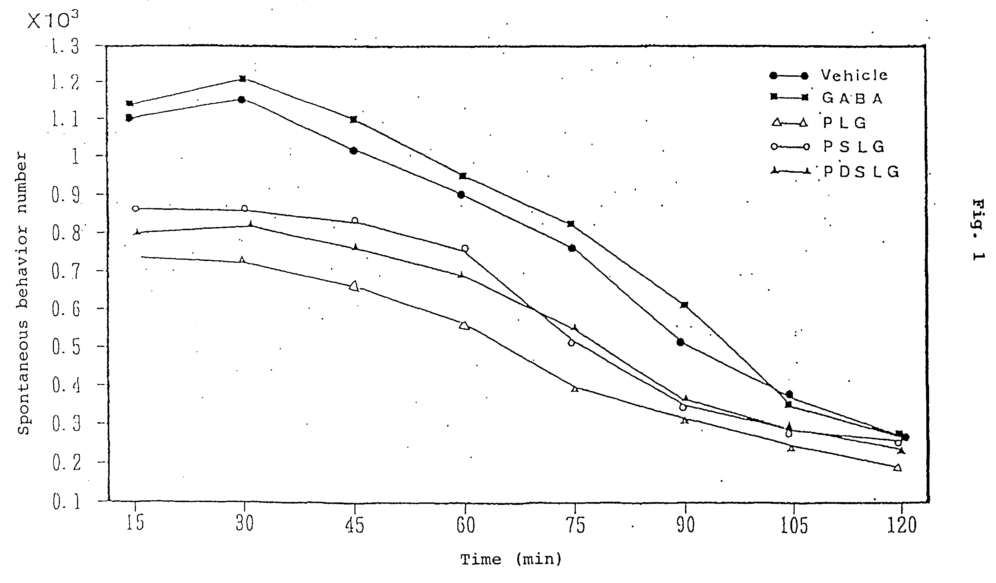

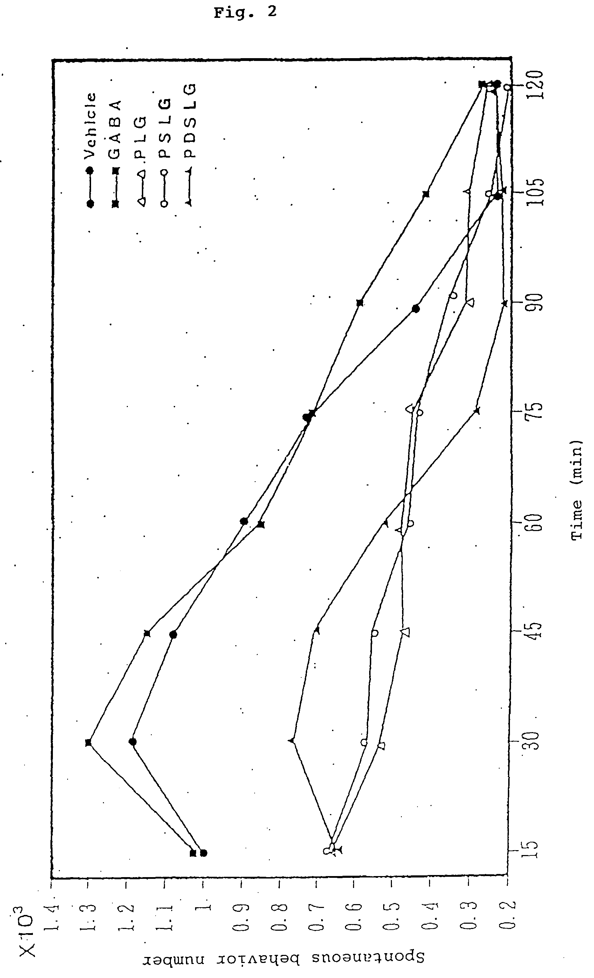

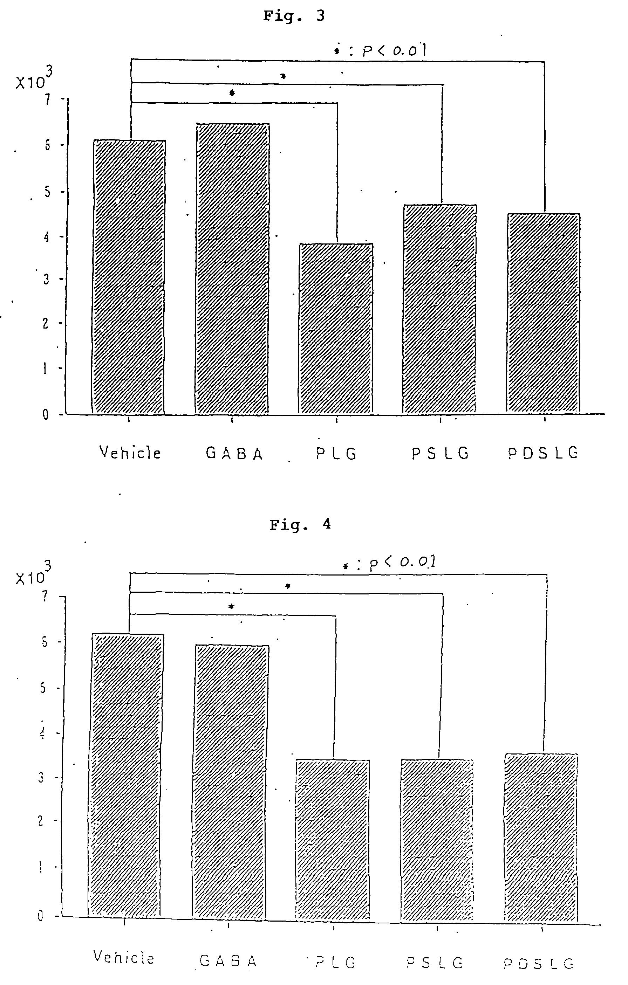

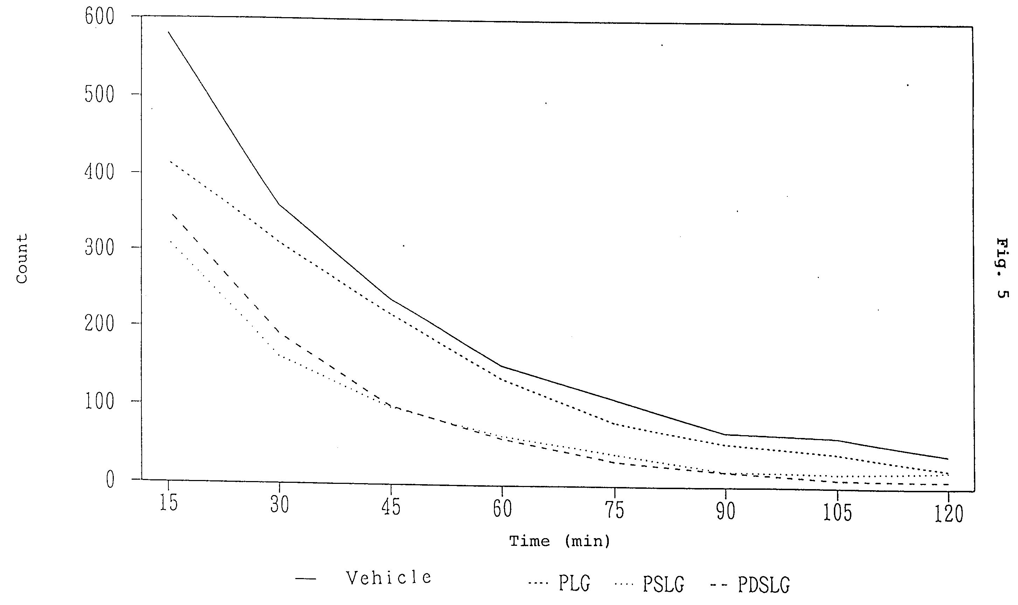

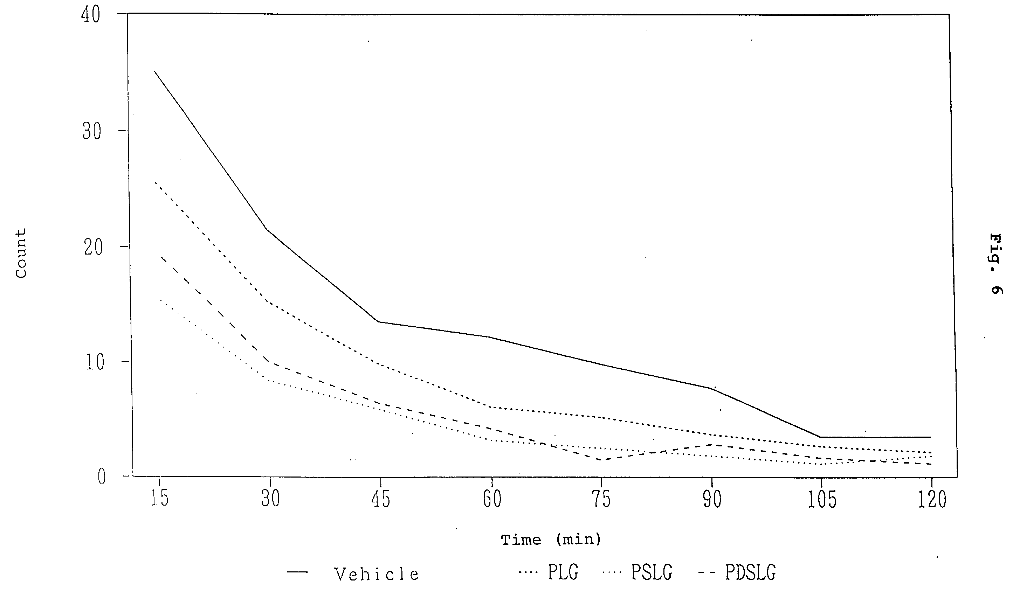

Fig. 1 shows spontaneous behavior inhibition action induced by methamphetamine when control or each drug is administered to rat tonsillar nucleus. The number of spontaneous behavior is plotted as ordinate and the time as abscissa. Fig. 2 shows spontaneous behavior inhibition action induced by methamphetamine when control or each drug is administered to rat caudate nucleus putamen. The number of spontaneous behavior is plotted as ordinate and the time as abscissa. Fig. 3 shows spontaneous behavior inhibition action induced by methamphetamine when control or each drug is administered to rat tonsillar nucleus in terms of the integration of spontaneous behavior numbers. Fig. 4 shows spontaneous behavior inhibition action induced by methamphetamine when control or each drug is administered to rat caudate nucleus putamen in terms of the integration of spontaneous behavior numbers. Fig. 5 shows spontaneous behavior inhibition action induced by Bay K8644 when control or each drug is administered to rat caudate nucleus putamen. The number of spontaneous behavior is plotted as ordinate and the time as abscissa. Fig. 6 shows rearing inhibition action induced by Bay K8644 when control or each drug is administered to rat caudate nucleus putamen. The number of rearing is plotted as ordinate and the time as abscissa.

## BEST MODE FOR PRACTICE OF THE INVENTION

Examples of the present invention are given below by way of illustration and not by way of limitation.

## Example 1

Production of Piv-L-Ser-Leu-GABA (hereinafter referred to as PSLG)

(1) Production of Boc-Leu-GABA

A 4.98 g portion of Boc-Leu $\cdot$ H$_2$O was dissolved in 30 ml of THF, 2.53 g of HONSu was dissolved in the resulting solution under cooling at 0°C, the thus prepared solution was mixed with 4.54 g of DCC which had been dissolved in advance in 15 ml of THF, and the mixture was stirred for 1 hour under ice-cooling and then overnight at room temperature. The reaction solution was mixed with 2.06 g of γ-aminobutyric acid which had been dissolved in 10 ml of water, and the mixture was adjusted to neutral pH with NMM and then stirred overnight at room temperature. The reaction mixture was concentrated under reduced pressure, and the resulting residue was mixed with 100 ml of water and 200 ml of ethyl acetate and then washed twice with 200 ml of 1 N hydrochloric acid and three times with 200 ml of a saturated sodium chloride solution in that order. This was dried over anhydrous magnesium sulfate and then concentrated under reduced pressure. The residual oily substance was subjected to column chromatography using 200 g of silica gel. During development of the substance with toluene-ethyl acetate (1:1), the compound of interest in the elution fractions was monitored by silica gel thin layer chromatography using benzene-ethyl acetate (1:1), and then the compound of interest was collected, dissolved in 250 ml of ethyl acetate and washed twice with a saturated sodium chloride solution. This was dried over anhydrous magnesium and then concentrated under reduced pressure. The resulting residue was treated with n-hexane to obtain Boc-Leu-GABA.

## (2) Production of Boc-L-Ser-Leu-GABA

Five ml of TFA was added to 2.35 g of Boc-Leu-GABA to dissolve the latter under ice-cooling, and the solution was stirred for 30 minutes and then concentrated under reduced pressure. The thus obtained residue was treated with ether, and the precipitated product was collected by filtration and dried *in vacuo* on sodium hydroxide to obtain Leu-GABA • TFA salt. A 2.1 g portion of Boc-L-Ser was dissolved in 30 ml of THF, 1.27 g of HONSu was dissolved in the resulting solution under cooling at 0°C, the thus prepared solution was mixed with 2.77 g of DCC which had been dissolved in advance in 10 ml of THF, and the mixture was stirred for 1 hour under ice-cooling and then overnight at room temperature. The reaction solution was mixed with 2.5 g of Leu-GABA • TFA salt which had been dissolved in 10 ml of water, and the mixture was adjusted to neutral pH with NMM and then stirred overnight at room temperature. The resulting residue was mixed with 400 ml of ethyl acetate and then washed twice with 200 ml of 1 N hydrochloric acid and three times with 200 ml of a saturated sodium chloride solution in that order.

This was dried over anhydrous magnesium sulfate and concentrated under reduced pressure to obtain Boc-Ser-Leu-GABA.

## (3) Production of PSLG

A 4.5 g portion of Boc-L-Ser-Leu-GABA was added to and dissolved in 5 ml of TFA under ice-cooling, and the solution was stirred for 30 minutes and then concentrated under reduced pressure. The thus obtained residue was treated with ether, and the precipitated product was collected by filtration and dried *in vacuo* over sodium hydroxide to obtain L-Ser-Leu-GABA • TFA salt.

A 3.5 g portion of the thus synthesized product was dissolved in 30 ml of THF and mixed with 1.3 g of pivaloyl chloride, and the mixture was adjusted to neutral pH with NMM and then stirred overnight at room temperature. The reaction mixture was concentrated under reduced pressure, and the resulting residue was mixed with 100 ml of water and 200 ml of ethyl acetate and then washed twice with 200 ml of 1 N hydrochloric acid and three times with 200 ml of a saturated sodium chloride solution in that order. This was dried over anhydrous magnesium sulfate and then concentrated under reduced pressure. The residual oily substance was subjected to column chromatography using 200 g of silica gel. During development of the substance with toluene-ethyl acetate (1:1), the compound of interest in the elution fractions was monitored by silica gel thin layer chromatography using benzene-ethyl acetate (1:1), and then the compound of interest was collected, dissolved in 250 ml of ethyl acetate and washed twice with a saturated sodium chloride solution. This was dried over anhydrous magnesium and then concentrated under reduced pressure to obtain PSLG.

m.p. 158°C

$[\alpha]_D^{20} = +16.8°$ (C = 1, H$_2$O)

| Elemental analysis for C$_{18}$H$_{33}$O$_6$N$_3$ | | | |
|---|---|---|---|
| calcd. | C (55.8%), | H (8.58%), | N (10.85%) |
| found | C (55.1%), | H (8.66%), | N (10.87%) |

Amino acid composition
L-Ser 0.87 (1), L-Leu 1.0 (1)

## Example 2

Production of Piv-D-Ser-Leu-GABA (hereinafter referred to as PDSLG)

The above compound was produced in accordance with the process of Example 1, except for using D-Ser in place of L-Ser.

m.p. 160°C

$[\alpha]_D^{20} = +7.7°$ (C = 1, H$_2$O)

| Elemental analysis for C$_{18}$H$_{33}$O$_6$N$_3$ | | | |
|---|---|---|---|
| calcd. | C (55.8%), | H (8.58%), | N (10.85%) |
| found | C (54.98%), | H (8.49%), | N (10.80%) |

Amino acid composition
D-Ser 0.80 (1), L-Leu 1.0 (1)

## Test Example 1

Wistar rats (250 to 300 g) were anesthetized with pentobarbital (50 mg/kg, i.p.) and their parietal regions were shaved off and the head was respectively fixed on a rat brain fixing apparatus. After incising the scalp, a hole of 1 to 1.2 mm was made in the cranial bones using an electric drill, and a stainless steel guide cannula of 1 to 1.2 mm in diameter and 23 mm in length was inserted into the hole and fixed to the cranial bones with dental cement. As the insertion position of the guide cannula, co-ordinates of tonsillar nucleus (A: -1.3 mm, L: 3.4 mm and V: 8.6 mm from bregma) were selected with reference to the brain illustrations of Paxions and Watson. Rats were reared for 1 week after the operation and then used in the test.

Each of the compounds to be tested (GABA, PLG, PSLG and PDSLG) dissolved in physiological saline containing 13% ethanol to give a concentration of 10 μmol/ml was administered from the previously fixed guide cannula for 2 minutes at a rate of 2.7 μl/min, and 5 minutes thereafter 1.0 mg/kg of methamphetamine was intraperitoneally administered to measure the number of spontaneous behavior every 15 minutes for 2 hours using Automex II (a spontaneous motion quantity measuring apparatus, manufactured by Columbus, U.S.A.).

Drug-free physiological saline containing 13% ethanol (vehicle) was used as a control in the same manner. Results of the measurement are shown in Fig. 1.

Test Example 2

The procedure of Test Example 1 was repeated except that co-ordinates of caudate nucleus putamen (A: 1.7 mm, L: 2.4 mm and V: 5.0 mm from bregma) were selected as the insertion position of the guide cannula with reference to the brain illustrations of Paxions and Watson. Results of the measurement are shown in Fig. 2.

In the tonsillar nucleus administration control group, the number of spontaneous behavior of rats reached the maximum value of about 1,250 counts 30 minutes after administration of methamphetamine. Though the number of spontaneous behavior gradually decreased thereafter, the number after 2 hours was still about 280 counts which were 8 times higher than the counts of about 30 before administration of methamphetamine. On the contrary, in a test group in which PLG or the peptide compound of the present invention was administered to tonsillar nucleus, inhibition of the number of spontaneous behavior after administration of methamphetamine became obvious after 15 minutes, and significant inhibition was observed after 30 minutes of the administration, with the number of about 900 counts by PSLG, about 820 counts by PDSLG and about 750 counts by PLG. Similar to the above case, significant inhibition effect was observed in the caudate nucleus putamen administration groups, with about 650 counts after 15 minutes in each group in which PLG or the peptide compound of the present invention was administered and about 600 counts after 30 minutes in each of the PLG- and PSLG-administered groups. After 75 minutes, PDSLG showed a considerable inhibition of about 300 counts, thus indicating site selectivity of PDSLG (stronger action on caudate nucleus putamen).

The total number of spontaneous behavior observed within 2 hours after administration of methamphetamine in the drug-administered group was compared with that in the control group. As shown in Fig. 3, the inhibition in the tonsillar administration group was about 35% by PLG and about 25% by PSLG and PDSLG. On the other hand, in the group in which PLG or the peptide compound of the present invention was administered to caudate nucleus putamen, both showed 45% or more inhibition as shown in Fig. 4.

In this connection, the inhibition effect was observed also by intracerebral administration of γ-aminobutyric acid alone, but 80 times or more larger dose was required to obtain the effect, in comparison with the dose of the peptide compound of the present invention.

Test Example 3

The operation of Test Example 1 was repeated except that male Wistar rats (300 to 350 g) were used and co-ordinates of caudate nucleus putamen (A: 1.7 mm, L: 2.4 mm and V: 5.0 mm from bregma) were selected as the insertion position of the guide cannula with reference to the brain illustrations of Paxions and Watson. Rats were reared for 1 week after the operation and then used in the test.

Each of the compounds to be tested (PLG, PSLG and PDSLG) dissolved in physiological saline containing 13% ethanol to give a concentration of 10 μmol/ml was administered from the previously fixed guide cannula for 2 minutes at a rate of 2.7 μl/min. Immediately after administration of each drug, (±) Bay K8644 (methyl 1,4-dihydro-2,6-dimethyl-5-nitro-4-(2-(trifluoromethyl)phenyl)-3-pyridinecarboxylate, manufactured by Wako Pure Chemical Industries) (18.5 μmol/ml) was administered for 2 minutes at a rate of 2.7 μl/min to measure the number of spontaneous behavior every 15 minutes for 2 hours using Automex II. At the same time, the number of rearing was counted. The term rearing as used herein means an action to stand upright solely with hind legs, which is counted by the naked eye.

Drug-free physiological saline containing 13% ethanol (vehicle) was used as a control in the same manner. The number of spontaneous behavior is shown in Fig. 5, and the number of rearing in Fig. 6, both induced by (±) Bay K8644.

In comparison with the control group, the number of spontaneous behavior was decreased in each of the PLG-, PSLG- and PDSLG-administered groups, and the inhibition action of PSLG and PDSLG was stronger than that of PLG.

With regard to the number of rearing, PSLG and PDSLG showed definitely strong inhibition effect in comparison with PLG.

In this model, actions of various drugs on the release of dopamine were examined by counting the rearing which is considered to be induced via dopamine, and it was indicated that PSLG and PDSLG are possessed of an effect to inhibit release of dopamine because of their significant effect to reduce the number of rearing in comparison with PLG.

INDUSTRIAL APPLICABILITY

As is evident from the above tests, the peptide compound of the present invention has an antispasmodic function which is markedly higher than γ-aminobutyric acid, and therefore, it can be used as a central nervous system depressant in the treatment of for example schizoid or epilepsy. In addition, it can keep homeostasis of the central nervous system by acting upon the release and metabolism of various neurotransmitters, as well as various receptors, cell membranes and the like, and functions as a psychopharmaceuticals for use in the treatment of dementia, improvement of cerebral circulation and the like.

## Claims

1. A peptide compound represented by the formula (1)

    R-CO-(L or D)-Ser-X-GABA        (1)

    wherein R-CO represents an alkylcarbonyl group, an alkoxycarbonyl group or an amino group-protecting group used in peptide chemistry; (L or D)-Ser represents an (L or D)-serine residue; GABA represents a $\gamma$-aminobutyric acid residue; and X represents a glycine residue or an L-leucine residue, and a salt thereof.

2. The peptide compound and a salt thereof according to claim 1 wherein said X is L-leucine residue.

3. A central nervous system depressant which comprises a peptide compound represented by the formula (1)

    R-CO-(L or D)-Ser-X-GABA        (1)

    wherein R-CO represents an alkylcarbonyl group, an alkoxycarbonyl group or an amino group-protecting group used in peptide chemistry; (L or D)-Ser represents an (L or D)-serine residue; GABA represents a $\gamma$-aminobutyric acid residue; and X represents a glycine residue or an L-leucine residue, or a pharmaceutically acceptable salt thereof as an active ingredient.

4. The central nervous system depressant according to claim 3 wherein said X is L-leucine residue.

Fig. 1

Fig. 2

Fig. 3

Fig. 4

Fig. 5

Fig. 6

Count

Time (min)

— Vehicle   ··· PLG   ···· PSLG   -- PDSLG

**INTERNATIONAL SEARCH REPORT**

International application No.

PCT/JP94/01394

| A. | CLASSIFICATION OF SUBJECT MATTER |
|---|---|

Int. Cl⁶   C07K5/027, A61K38/05

According to International Patent Classification (IPC) or to both national classification and IPC

| B. | FIELDS SEARCHED |
|---|---|

Minimum documentation searched (classification system followed by classification symbols)

Int. Cl⁵   C07K5/02, 5/06, A61K37/02

Documentation searched other than minimum documentation to the extent that such documents are included in the fields searched

Electronic data base consulted during the international search (name of data base and, where practicable, search terms used)

CAS ONLINE

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP, A, 64-52744 (Nippon Zoki Pharmaceutical Co., Ltd.), February 28, 1989 (28. 02. 89), Claim, line 3, upper left column to line 9, lower right column, page 3, (Family: none) | 1-4 |
| A | JP, A, 62-270552 (Nippon Zoki Pharmaceutical Co., Ltd.), November 24, 1987 (24. 11. 87), Claim, line 3, upper left column to line 9, lower right column, page 3 & EP, A2, 221019 & US, A, 5110797 | 1-4 |

| ☐ | Further documents are listed in the continuation of Box C. | ☐ | See patent family annex. |
|---|---|---|---|

| * | Special categories of cited documents: |
|---|---|
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| October 31, 1994 (31. 10. 94) | November 29, 1994 (29. 11. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)